Europäisches Patentamt

European Patent Office

Office européen des brevets

(19)

(11) EP 0 407 622 B1

(12) EUROPEAN PATENT SPECIFICATION

(45) Date of publication and mention
of the grant of the patent:
**04.12.1996 Bulletin 1996/49**

(21) Application number: **90902684.1**

(22) Date of filing: **01.02.1990**

(51) Int Cl.6: **C07C 19/10**, C07C 17/20

(86) International application number:
**PCT/JP90/00123**

(87) International publication number:
**WO 90/08754 (09.08.1990 Gazette 1990/19)**

(54) **PROCESS FOR PRODUCING A 2,2-DIFLUOROPROPANE**

VERFAHREN ZUR HERSTELLUNG EINES 2,2-DIFLUORPROPANS

PROCEDE DE PRODUCTION D'UN 2,2-DIFLUOROPROPANE

(84) Designated Contracting States:
**DE FR GB IT NL**

(30) Priority: **02.02.1989 JP 122550/89**
**02.02.1989 JP 122621/89**
**03.02.1989 JP 23741/89**
**03.02.1989 JP 123742/89**
**03.02.1989 JP 123743/89**
**03.02.1989 JP 23744/89**
**03.02.1989 JP 123745/89**
**03.02.1989 JP 123750/89**
**06.02.1989 JP 125653/89**
**06.02.1989 JP 125654/89**
**06.02.1989 JP 125655/89**
**06.02.1989 JP 125656/89**
**06.02.1989 JP 25657/89**
**06.02.1989 JP 125658/89**
**06.02.1989 JP 125681/89**

(43) Date of publication of application:
**16.01.1991 Bulletin 1991/03**

(73) Proprietor: **ASAHI GLASS COMPANY LTD.**
**Chiyoda-ku Tokyo 100 (JP)**

(72) Inventors:
• **MORIKAWA, Shinsuke**
**Yokohama-shi Kanagawa-ken 236 (JP)**
• **SAMEJIMA, Shunichi**
**Tokyo 164 (JP)**
• **OKAMOTO, Hidekazu**
**Yokohama-shi Kanagawa-ken 241 (JP)**
• **OHNISHI, Keiichi**
**Yokohama-shi Kanagawa-ken 221 (JP)**
• **TATEMATSU, Shin**
**Tokyo 160 (JP)**
• **TANUMA, Toshihiro**
**Yokohama-shi Kanagawa-ken 233 (JP)**
• **OHMORI, Takashi**
**Tokyo 152 (JP)**

(74) Representative:
**Wächtershäuser, Günter, Prof. Dr.**
**Patentanwalt,**
**Tal 29**
**80331 München (DE)**

(56) References cited:
**EP-A- 0 317 981        GB-A- 938 070**
**GB-A- 945 017         GB-A- 975 498**
**GB-A- 999 444         US-A- 2 490 764**
**US-A- 2 578 721**

• **Houben-Weyl: "Methoden der Organischen Chemie, 4th Edition, Volume V/3, Halogenverbindungen", 1962, Georg Thieme Verlag, (Stuttgart, DE), see pages 124-125**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

As a synthetic route for a 2,2-difluoropropane, a method has been known which comprises adding dichlorodifluoromethane or trichlorofluoromethane to an ethylene having a difluoromethylene unit, such as 1,1-dichloro-2,2-difluoroethylene or 1-chloro-1,2,2-trifluoroethylene, in the presence of aluminum chloride.

However, such a method has a drawback that it produces not only the desired product but also a reaction by-product having a methylene group other than the 2,2-difluoromethylene and having a boiling point close to that of the desired product, whereby a multi-stage purification process is required in order to obtain a product of a high purity. Further, a method for synthesizing 1-chloro-1,1,2,2-tetrafluoropropane is known which comprises fluorinating-propyne ($CH_3C{\equiv}CH$) with hydrogen fluoride to obtain 2,2-difluoropropane ($CH_3CF_2CH_3$), then selectively chlorinating only three hydrogen atoms at 1-position with chlorine to obtain 1,1,1-trichloro-2,2-difluoropropane ($CCl_3CF_2CH_3$), and further selectively fluorinating only two out of the three chlorine atoms substituted on the carbon atom at the 1-position to obtain 1-chloro-1,1,2,2-tetrafluoropropane (J. Am. Chem. Soc., 65, 2342 (1943)). This method requires many steps and thus has a drawback that it is difficult to improve the yield, and the method is not suitable for industrial production.

Further, a method for preparing 1-chloro-2,2,3,3-tetrafluoropropane is known which comprises producing 2,2,3,3-tetrafluoropropanol from tetrafluoroethylene and methanol, then reacting it with sulfuryl chloride to obtain a chlorosulfonic acid ester, and then reacting it with an alkali metal chloride. This method requires many steps, whereby it is difficult to improve the yield, and thus has a drawback that such a method is not suitable for industrial production.

Further, a method for preparing 1-chloro-2,2,3-trifluoropropane is known which comprises dehydrochlorinating 1,2,3-trichloropropane to obtain 2,3-dichloropropene, reacting it with potassium fluoride to obtain 2-chloro-3-fluoropropene, then adding chlorine thereto to obtain 1,2,2-trichloro-3-fluoropropane, and then fluorinating it with antimony dichloride trifluoride. Such a method requires many steps. Therefore, the method has a drawback that it is difficult to improve the yield. As such, the method is not suitable for industrial production.

DISCLOSURE OF THE INVENTION

The present inventors have conducted extensive researches for a process of effectively producing a 2,2-difluoropropane and, as a result, they have found it possible to obtain a 2,2-difluoropropane of the following formula (2) in good yield by fluorinating a chlorine-containing 2,2-halogenopropane of the formula (1) with hydrogen fluoride or a fluorinating agent. The present invention is based on this discovery.

The present invention provides a process as defined in claim 1.

The 2,2-difluoropropane is useful as an etching agent for the production of electronic parts. Further, a chlorine-containing 2,2-difluoropropane is expected to be useful as a foaming agent, a cooling medium, a propellant or a solvent like conventional chlorofluorocarbons. Particularly, it is expected to be useful as a solvent which can be a substitute for 1,1,2-trichlorotrifluoroethane. Further, it hardly depletes the ozone layer in the stratosphere. It is also useful as an intermediate for the production of a hydrogen-2,2-difluoropropane containing no chlorine.

BEST MODE OF CARRYING OUT THE INVENTION

Now, the present invention will be described in detail with reference to the preferred embodiments.

The fluorination in the present invention is preferably conducted by hydrogen fluoride in the presence of a fluorination catalyst.

The pressure for the reaction is not particularly limited, but it is usually within a range of from 0 to 30 kg/cm$^2$.

To prepare the catalysts, any method may be-employed so long as it is capable of uniformly dispersing the halides or oxides containing at least one element selected from the group consisting of the above ten elements. For example, a coprecipitation method or a kneading method may be mentioned. Particularly preferred is a method in which hydrates are co-precipitated from an aqueous solution of salts of the above metal elements, or a method wherein a cake of hydroxides is kneaded and pulverized by a ball mill or by a homogenizer. The hydroxides may be those precipitated from aqueous solutions of inorganic salts such as nitrates or sulfates by means of aqueous ammonia or urea, or those prepared by the hydrolysis of organic salts.

A catalyst in the state of a hydrate is preferably dried at a temperature of from 129 to 150°C, followed by calcining at a temperature of from 300 to 600°C, preferably from 350 to 450°C.

It is preferred to activate the catalyst. Such activation can be accomplished usually by applying fluorinating treatment at a temperature of from 100 to 450°C, preferably from 200 to 350°C. Otherwise, such activation can be conducted in the fluorination reaction system, or by heat treatment together with a fluorinated hydrocarbon.

It is preferred to conduct the fluorination reaction in a gas phase under atmospheric pressure or under pressurization at a temperature within a range of from 150 to 550°C, more preferably from 250 to 450°C.

The ratio between hydrogen fluoride and the chlorine-containing 2,2-halogenopropane starting material, may be varied in a wide range. However, in order to substitute the chlorine atoms, it is usual to use a stoichiometric amount of hydrogen fluoride. However, hydrogen fluoride may be used in an amount substantially larger than the stoichiometrical amount, for example, 4 mol times or more, relative to the total molar amount of the starting material. The contact time is usually from 0.1 to 300 seconds, preferably from 5 to 30 seconds.

To maintain the catalytic activity, oxygen or chlorine is preferably added in an amount of from 0.1 to 10% by volume relative to the chlorine-containing 2,2-halogenopropane starting material.

The following reactions (3) to (7) may be mentioned as specific embodiments for producing a 2,2-difluoropropane of the formula (2)

$$C_3Cl_{6-m^1}F_{2+m^1} \rightarrow C_3Cl_{6-n^1}F_{2+n^1} \qquad (3)$$

$0 \leqq m^1 \leqq 5$  $1 \leqq n^1 \leqq 6$, $m^1 < n^1$

The chlorine-containing 2,2-halogenopropane ($C_3Cl_{6-m^1}F_{2+m^1}$ wherein $0 \leqq m^1 \leqq 5$) to be used as the starting material includes 1,1,1,3,3,3-hexachloro-2,2-difluoropropane (R-212ca), 1,1,1,3,3-pentachloro-2,2,3-trifluoropropane (R-213ca), 1,1,3,3-tetrachloro-1,2,2,3-tetrafluoropropane (R-214ca), 1,1,1,3-tetrachloro-2,2,3,3-tetrafluoropropane (R-214cb), 1,1,3-trichloro-1,2,2,3,3-pentafluoropropane (R-215ca), 1,1,1-trichloro-2,2,3,3,3-pentafluoropropane (R-215cb), 1,3-dichloro-1,1,2,2,3,3-hexafluoropropane (R-216ca), 1,1-dichloro-1,2,2,3,3,3-hexafluoropropane (R-216cb) and 1-chloro-1,1,2,2,3,3,3-heptafluoropropane (R-217ca).

The 2,2-difluoropropane ($C_3Cl_{6-n^1}F_{2+n^1}$ wherein $1 \leqq n^1 \leqq 6$) to be formed by the reaction includes 1,1,1,3,3-pentachloro-2,2,3-trifluoropropane (R-213ca), 1,1,3,3-tetrachloro-1,2,2,3-tetrafluoropropane (R-214ca), 1,1,1,3-tetrachloro-2,2,3,3-tetrafluoropropane (R-214cb), 1,1,3-trichloro-1,2,2',3,-3-pentafluoropropane (R-215ca), 1,1,1-trichloro-2,2,3,3,3-pentafluoro-propane (R-215cb), 1,3-dichloro-1,1,2,2,3,3-hexafluoropropane (R-216ca), 1,1-dichloro-1,2,2,3,3,3-hexafluoropropane (R-216cb), 1-chloro-1,1,2,2,3,3,3-heptafluoropropane (R-217ca) and octafluoropropane (R-218). These products can be separated by a usual method such as distillation.

$$C_3HCl_{5-m^2}F_{2+m^2} \rightarrow C_3HCl_{5-n^2}F_{2+n^2} \qquad (4)$$

$0 \leqq m^2 \leqq 4$  $1 \leqq n^2 \leqq 5$, $m^2 < n^2$

The chlorine-containing 2,2-halogenopropane ($C_3HCl_{5-m^2}F_{2+m^2}$ wherein $0 \leqq m^2 \leqq 4$) to be used as the starting material includes 1,1,1,3,3-pentachloro-2;2-difluoropropane (R-222ca), 1,1,3,3-tetrachloro-1,2,2-trifluoropropane (R-223ca), 1,1,1,3-tetrachloro-2,2,3-trifluoropropane (R-223cb), 1,1,3-trichloro-2,2,3,3-tetrafluoropropane (R-224ca), 1,1,3-trichloro-1,2,2,3-tetrafluoropropane (R-224cb), 1,1,1-trichloro-2,2,3,3-tetrafluoropropane (R-224cc), 1,1-dichloro-2,2,3,3,3-pentafluoropropane (R-225ca), 1,3-dichloro-1,1,2,2,3-pentafluoropropane (R-225cb), 1,1-dichloro-1,2,2,3,3-pentafluoropropane (R-225cc), 1-chloro-1,2,2,3,3,3-hexafluoropropane (R-226ca) and 1-chloro-1,1,2,2,3,3-hexafluoropropane (R-226cb). The 2,2-difluoropropane ($C_3HCl_{5-n^2}F_{2+n^2}$ wherein $1 \leqq n^2 \leqq 5$) to be formed by the reaction includes 1,1,3,3-tetrachloro-1,2,2-trifluoropropane (R-223ca), 1,1,1,3-tetrachloro-2,2,3-trifluoropropane (R-223cb), 1,1,3-trichloro-2,2,3,3-tetrafluoropropane (R-224ca), 1,1,3-trichloro-1,2,2,3-tetrafluoropropane (R-224cb)-, 1,1,1-trichloro-2,2,3,3-tetrafluoropropane (R-224cc), 1,1-dichloro-2,2,3,3,3-pentafluoropropane (R-225ca), 1,3-dichloro-1,1,2,2,3-pentafluoropropane (R-225cb), 1,1-dichloro-1,2,2,3,3-pentafluoropropane (R-225cc), 1-chloro-1,2,2,3,3,3-hexafluoropropane (R-226ca), 1-chloro-1,1,2,2,3,3-hexafluoropropane (R-226cb) and 1,1,1,2,2,3,3-heptafluoropropane (R-227ca). These products can be separated by a usual method such as distillation.

$$C_3H_2Cl_{4-m^3}F_{2+m^3} \rightarrow C_3H_2Cl_{4-n^3}F_{2+n^3} \qquad (5)$$

$0 \leqq m^3 \leqq 3$  $1 \leqq n^3 \leqq 4$, $m^3 < n^3$

The chlorine-containing 2,2-halogenopropane ($C_3H_2Cl_{4-m^3}F_{2+m^3}$ wherein $0 \leqq m^3 \leqq 3$) to be used as the starting material includes 1,1,1,3-tetrachloro-2,2-difluoropropane (R-232cb), 1,1,3,3-tetrachloro-2,2-difluoropropane (R-232ca), 1,1,3-trichloro-1,2,2-trifluoropropane (R-233cb), 1,1-dichloro-1,2,2,3-tetrafluoropropane (R-234cd), 1,3-dichloro-1,1,2,2-tetrafluoropropane (R-234cc), 1,1-dichloro-2,2,3,3-tetrafluoropropane (R-234cb), 1,3-dichloro-1,2,2,3-tetrafluoropropane (R-234ca), 1-chloro-1,1,2,2,3-pentafluoropropane (R-235cc), 3-chloro-1,1,1,2,2-pentafluoropropane (R-235cb) and 1-chloro-1,2,2,3,3-pentafluoropropane (R-235ca).

The 2,2-difluoropropane ($C_3H_2Cl_{4-n^3}F_{2+n^3}$ wherein $1 \leqq n^3 \leqq 4$) to be formed by the reaction includes 1,1,1-trichloro-2,2,3-trifluoropropane (R-233cc), 1,1,3-trichloro-1,2,2-trifluoropropane (R-233cb), 1,1,3-trichloro-2,2,3-trifluoropropane (R-233ca), 1,1-dichloro-1,2,2,3-tetrafluoropropane (R-234cd), 1,3-dichloro-1,1,2,2-tetrafluoropropane (R-234cc), 1,1-dichloro-2,2,3,3-tetrafluoropropane (R-234cb), 1,3-dichloro-1,2,2,3-tetrafluoropropane (R-234ca), 1-chloro-1,1,2,2,3-pentafluoropropane (R-235cc), 3-chloro-1,1,1,2,2-pentafluoropropane (R-235cb), 1-chloro-1,2,2,3,3-pentafluoropropane (R-235ca), 1,1,1,2,2,3-hexafluoropropane (R-236cb) and 1,1,2,2,3,3-hexafluoropropane (R-236ca). These products can be separated by a usual method such as distillation.

$$C_3H_3Cl_{3-m^4}F_{2+m^4} \rightarrow C_3H_3Cl_{3-n^4}F_{2+n^4} \qquad (6)$$

$0 \leqq m^4 \leqq 2 \qquad 1 \leqq n^4 \leqq 3, m^4 < n^4$

The chlorine-containing 2,2-halogenopropane ($C_3H_3Cl_{3-m^4}F_{2+m^4}$ wherein $0 \leqq m^4 \leqq 2$) to be used as the starting material includes 1,1,3-trichloro-2,2-difluoropropane (R-242ca), 1,1,1-trichloro-2,2-difluoropropane (R-242cb), 1,3-dichloro-1,2,2-trifluoropropane (R-243ca), 1,1-dichloro-2,2,3-trifluoropropane (R-243cb), 1,1-dichloro-1,2,2-trifluoropropane (R-243cc), 1-chloro-2,2,3,3-tetrafluoropropane (R-244ca), 1-chloro-1,2,2,3-tetrafluoropropane (R-244cb) and 1-chloro-1,1,2,2-tetrafluoropropane (R-244cc).

The 2,2-difluoropropane and trihydrochlorofluoropropane ($C_3H_3Cl_{3-n^4}F_{2+n^4}$ wherein $1 \leqq n^4 \leqq 3$) to be formed by the reaction include 1,3-dichloro-1,2,2-trifluoropropane (R-243ca), 1,1-dichloro-2,2,3-trifluoropropane (R-243cb), 1,1-dichloro-1,2,2-trifluoropropane (R-243cc), 1-chloro-2,2,3,3-tetrafluoropropane (R-244ca), 1-chloro-1,2,2,3-tetrafluoropropane (R-244cb), 1-chloro-1,1,2,2-tetrafluoropropane (R-244cc), 1,1,2,2,3-pentafluoropropane (R-245ca) and 1,1,1,2,2-pentafluoropropane (R-245cb). These products can be separated by a usual method such as distillation.

$$C_3H_4Cl_{2-m^5}F_{2+m^5} \rightarrow C_3H_4Cl_{2-n^5}F_{2+n^5} \qquad (7)$$

$0 \leqq m^5 \leqq 1 \qquad 1 \leqq n^5 \leqq 2, m^5 < n^5$

The chlorine-containing 2,2-halogenopropane ($C_3H_4Cl_{2-m^5}F_{2+m^5}$ wherein $0 \leqq m^5 \leqq 1$) to be used as the starting material includes 1,3-dichloro-2,2-difluoropropane (R-252ca), 1,1-dichloro-2,2-difluoropropane (R-252cb), 1-chloro-2,2,3-trifluoropropane (R-253ca) and 1-chloro-1,2,2-trifluoropropane (R-253cb).

The 2,2-difluoropropane ($C_3H_4Cl_{2-n^5}F_{2+n^5}$ wherein $1 \leqq n^5 \leqq 2$) to be formed by the reaction includes 1-chloro-2,2,3-trifluoropropane (R-253ca), 1-chloro-1,2,2-trifluoropropane (R-253cb), 1,2,2,3-tetrafluoropropane (R-254ca) and 1,1,2,2-tetrafluoropropane (R-254cb). These products can be separated by a usual method such as distillation. taking a long period of time-are taken into account, the following reactions (8) to (12) may be mentioned.

$$C_3Cl_{8-m^6}F_{m^6} \rightarrow C_3Cl_{8-n^6}F_{n^6} \qquad (8)$$

$0 \leqq m^6 \leqq 5 \qquad 2 \leqq n^6 \leqq 8, m^6 < n^6$

The chlorine-containing 2,2-halogenopropane ($C_3Cl_{8-m^6}F_{m^6}$ wherein $0 \leqq m^6 \leqq 5$) to be used as the starting material includes 1,1,1,2,2,3,3,3-octachloropropane (R-210aa), 1,1,1,2,3,3,3-heptachloro-2-fluoropropane (R-211ba), 1,1,1,3,3,3-hexachloro-2,2-difluoropropane (R-212ca), 1,1,1,3,3-pentachloro-2,2,3-trifluoropropane (R-213ca), 1,1,3,3-tetrachloro-1,2,2,3-tetrafluoorpropane (R-214ca), 1,1,1,3-tetrachloro-2,2,3,3-tetrafluoropropane (R-214cb).

Now, the present invention will be described in further detail with reference to Examples. However, it should be understood that the present invention is by no means restricted by such specific Examples.

PREPARATION EXAMPLE 1

1,200 g of $Cr(NO_3)_3 \cdot 9H_2O$ and 100 g of $Mg(NO_3)_2 \cdot 6H_2O$ were dissolved in 2.5 $\ell$ of water, and this solution and 2,000 g of 28% ammonium hydroxide aqueous solution were added to 4 $\ell$ of hot water under stirring to give precipitates of the hydroxides. The precipitates were collected by filtration, washed with pure water, dried and then sintered at 450°C for 5 hours to give the powder of the oxides. The powder was shaped into a cylindrical tablet having a diameter of 5 mm and a height of 5 mm by a tabletting machine. The catalyst thereby obtained was activated by fluorinating at a temperature of from 250 to 400°C in a gas stream of hydrogen fluoride/nitrogen mixture before using it in the reaction.

PREPARATION EXAMPLE 2

1,100 g of guaranteed reagent of $Al(NO_3)_3 \cdot 9H_2O$, 125 g of $Cr(NO_3)_3 \cdot 9H_2O$ and 40 g of $Mg(NO_3)_2 \cdot 6H_2O$, were dissolved in 2.5 $\ell$ of water, and this solution and 2,000 g of 28% ammonium hydroxide aqueous solution were added

EP 0 407 622 B1

to 4 ℓ of hot water to give precipitates of the hydroxides. The precipitates were collected by filtration, washed with pure water, dried and sintered at 450°C for 5 hours to give the powder of the oxides. The powder was shaped into a cylindrical tablet having a diameter of 5 mm and a height of 5 mm by a tabletting machine. The catalyst thereby obtained was activated by fluorinating at a temperature of from 250 to 400°C in a gas stream of hydrogen fluoride/nitrogen mixture before using it in the reaction.

PREPARATION EXAMPLES 3 TO 6

The catalysts were prepared in the same manner as in Preparation Example 2 except that 40 g of $Ba(NO_3)_2$, 50 g of $Sr(NO_3)_2$, 40 g of $Ca(NO_3)_2 \cdot 4H_2O$ and 60 g of $Mn(NO_3)_2 \cdot 4H_2O$ were used in place of $Mg(NO_3)_2 \cdot 6H_2O$, respectively.

PREPARATION EXAMPLE 7

The catalyst was prepared in the same manner as in Preparation Example 2 except that 300 g of $Fe(NO_3)_2 \cdot 9H_2O$ and 900 g of $Al(NO_3)_3 \cdot 9H_2O$ were used in place of $Al(NO_3)_3 \cdot 9H_2O$, $Cr(NO_3)_3 \cdot 9H_2O$ and $Mg(NO_3)_2 \cdot 6H_2O$.

PREPARATION EXAMPLE 8

The catalyst was prepared in the same manner as in Preparation Example 2 except that 600 g of $Fe(NO_3)_2 \cdot 9H_2O$ and 150 g of $Cr(NO_3)_3 \cdot 9H_2O$ were used in place of $Al(NO_3)_3 \cdot 9H_2O$, $Cr(NO_3)_3 \cdot 9H_2O$ and $Mg(NO_3)_2 \cdot 6H_2O$.

PREPARATION EXAMPLE 9 (Reference)

200 g of $AlCl_3$ was dissolved in 2 ℓ of water. To this solution, 1,000 g of commercially available γ-alumina was added, and then it was dried to remove moisture. Further, the catalyst thereby obtained was activated by the same activating method as in Preparation Example 1.

PREPARATION EXAMPLE 10 (Reference)

The catalyst was prepared in the same manner as in Preparation Example 9 except that 200 g of $CrCl_3 \cdot 6H_2O$ was used in place of $AlCl_3$. Further, the catalyst thereby obtained was activated by the same activating method as in Preparation Example 1.

PREPARATION EXAMPLE 11 (Reference)

The catalyst was prepared in the same manner as in Preparation Example 9 except that 200 g of $MnCl_2 \cdot 4H_2O$ was used in place of $AlCl_3$. Further, the catalyst thereby obtained was activated by the same activating method as in Preparation Example 1.

PREPARATION EXAMPLE 12

The catalyst was prepared in the same manner as in Preparation Example 9 except that 200 g of $NiCl_2 \cdot 6H_2O$ was used in place of $AlCl_3$. Further, the catalyst thereby obtained was activated by the same activating method as in Preparation Example 1.

PREPARATION EXAMPLE 13

The catalyst was prepared in the same manner as in Preparation Example 9 except that 200 g of $CoCl_2 \cdot 6H_2O$ was used in place of $AlCl_3$. Further, the catalyst thereby obtained was activated by the same activating method as in Preparation Example 1.

PREPARATION EXAMPLE 14 (Reference)

The catalyst was prepared in the same manner as in Preparation Example 9 except that 1,000 g of commercial available granulated activated carbon useful as a support for the catalyst was used in place of γ-alumina. Further, the catalsyt thereby obtained was activated by the same activating method as in Preparation Example 1.

5

EXAMPLE 6-1

An Inconnel 600 U-shaped reaction tube having an inner diameter of 2.54 cm and a length of 100 cm packed with 200 ml of fluorination catalyst prepared in the same manner as in Preparation Example 1, was used as a fluorination reactor. To the reactor kept at a temperature of 350°C, gasified 1,1,1-trichloropentafluoropropane, oxygen and hydrogen fluoride were fed in at a rate of 50 ml/minutes, 2 ml/minutes and 100 ml/minutes, respectively and reacted. The reaction products were collected in a trap cooled at -78°C. The gas composition, after removing acidic components from the collected product, was analyzed by gas chromatography and by $^{19}$F-NMR. The results are shown in Table 6-1.

EXAMPLE 6-2

The fluorination reaction and the analysis of the reaction products were conducted in the same manner as in Example 6-1 except that the catalyst prepared in
Preparation Example 2 was used. The results are shown in Table 6-1.

EXAMPLE 6-3

The fluorination reaction and the analysis of the reaction products were conducted in the same manner as in Example 6-1 except that the catalyst prepared in Preparation Example 3 by using $Ba(NO_3)_2$ was used. The results are shown in Table 6-1.

Table 6-1

| Example No. | 6-1 | 6-2 | 6-3 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 82 | 67 | 52 |
| Selectivity (%) $CF_3CF_2CCl_2F$ $CF_3CF_2CClF_2$ Others | 24 67 9 | 32 61 7 | 38 55 7 |

EXAMPLE 6-4

The fluorination reaction and the analysis of the reaction products were conducted in the same manner as in Example 6-1 except that the catalyst prepared in Preparation Example 4 by using $Sr(NO_3)_2$ was used. The results are shown in Table 6-2.

EXAMPLE 6-5

The fluorination reaction and the analysis of the reaction products were conducted in the same manner as in Example 6-1 except that the catalyst prepared in Preparation Example 5 by using $Ca(NO_3)_2 \cdot 4H_2O$ was used. The results are shown in Table 6-2.

EXAMPLE 6-6

The fluorination reaction and the analysis of the reaction products were conducted in the same manner as in Example 6-1 except that the catalyst prepared in Preparation Example 6 by using $Mn(NO_3)_2 \cdot 4H_2O$ was used. The results are shown in Table 6-2.

Table 6-2

| Example No. | 6-4 | 6-5 | 6-6 |
|---|---|---|---|
| Reaction temp. (°C) | 300 | 300 | 300 |

Table 6-2   (continued)

| Example No. | 6-4 | 6-5 | 6-6 |
|---|---|---|---|
| Ratio of HF/starting material (molar ratio) | 3 | 3 | 3 |
| Contact time (sec) | 20 | 20 | 20 |
| Conversion (%) | 44 | 50 | 70 |
| Selectivity (%) $CF_3CF_2CCl_2F$ $CF_3CF_2CClF_2$ Others | 34 58 8 | 40 50 10 | 29 64 7 |

EXAMPLE 6-7

The fluorination reaction and the analysis of the reaction products were conducted in the same manner as in Example 6-1 except that the catalyst prepared in Preparation Example 7 was used. The results are shown in Table 6-3.

EXAMPLE 6-8

The fluorination reaction and the analysis of the reaction products were conducted in the same manner as in Example 6-1 except that the catalyst prepared in Preparation Example 8 was used. The results are shown in Table 6-3.

EXAMPLE 6-9

The fluorination reaction and the analysis of the reaction products were conducted in the same manner as in Example 6-1 except that the catalyst prepared in Preparation Example 9 was used. The results are shown in Table 6-3.

Table 6-3

| Example No. | 6-7 | 6-8 | 6-9* |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 38 | 41 | 37 |
| Selectivity (%) $CF_3CF_2CCl_2F$ $CF_3CF_2CClF_2$ Others | 44 47 9 | 48 45 7 | 46 50 4 |

* Reference Example

EXAMPLE 6-10

The fluorination reaction and the analysis of the reaction products were conducted in the same manner as in Example 6-1 except that the catalyst prepared in Preparation Example 10 was used. The results are shown in Table 6-4.

EXAMPLE 6-11

The fluorination reaction and the analysis of the reaction products were conducted in the same manner as in Example 6-1 except that the catalyst prepared in Preparation Example 11 was used. The results are shown in Table 6-4.

EXAMPLE 6-12

The fluorination reaction and the analysis of the reaction products were conducted in the same manner as in Example 6-1 except that the catalyst prepared in Preparation Example 12 was used. The results are shown in Table 6-4.

Table 6-4

| Example No. | 6-10* | 6-11 | 6-12 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 38 | 41 | 37 |
| Selectivity (%)<br>$CF_3CF_2CCl_2F$<br>$CF_3CF_2CClF_2$<br>Others | 44<br>47<br>9 | 48<br>45<br>7 | 46<br>50<br>4 |

* Reference Ex.

EXAMPLE 6-13

The fluorination reaction and the analysis of the reaction products were conducted in the same manner as in Example 6-1 except that the catalyst prepared in Preparation Example 13 was used. The results are shown in Table 6-5.

EXAMPLE 6-14

The fluorination reaction and the analysis of the reaction products were conducted in the same manner as in Example 6-1 except that the catalyst prepared in Preparation Example 14 was used. The results are shown in Table 6-5.

Table 6-5

| Example No. | 6-13 | 6-14* |
|---|---|---|
| Reaction temp. (°C) | 300 | 300 |
| Ratio of HF/starting material (molar ratio) | 3 | 3 |
| Contact time (sec) | 20 | 20 |
| Conversion (%) | 38 | 41 |
| Selectivity (%)<br>$CF_3CF_2CCl_2F$<br>$CF_3CF_2CClF_2$<br>Others | 44<br>47<br>9 | 48<br>45<br>7 |

* Reference Ex.

EXAMPLE 6-15

The fluorination reaction and the analysis of the reaction products were conducted in the same manner as in Example 6-1 except that 2,2-difluoro-hexachloropropane was used. The results are shown in Table 6-6.

EXAMPLE 6-16

The fluorination reaction and the analysis of the reaction products were conducted in the same manner as in Example 6-1 except that 1,1,1,3,3-pentachlorotrifluoropropane was used. The results are shown in Table 6-6.

EXAMPLE 6-17

The fluorination reaction and the analysis of the reaction products were conducted in the same manner as in Example 6-1 except that 1,1,3,3-tetrachlorotetrafluoropropane was used. The results are shown in Table 6-6.

Table 6-6

| Example No. | 6-15 | 6-16 | 6-17 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 68 | 72 | 87 |
| Selectivity (%)<br>$CCl_3CF_2CClF_2$<br>$CCl_2FCF_2CCl_2F$<br>$CCl_3CF_2CF_3$<br>$CCl_2FCF_2CClF_2$<br>$CCl_2FCF_2CF_3$<br>$CClF_2CF_2CClF_2$<br>Others | <br>17<br>6<br><br>37<br>10<br>23<br>7 | <br>6<br>14<br>8<br>24<br>7<br>33<br>8 | <br><br><br><br>24<br>15<br>54<br>7 |

EXAMPLE 6-18

The fluorination reaction and the analysis of the reaction products were conducted in the same manner as in Example 6-1 except that 1,1,1,3-tetrachlorotetrafluoropropane was used. The results are shown in Table 6-7.

EXAMPLE 6-19

The fluorination reaction and the analysis of the reaction products were conducted in the same manner as in Example 6-1 except that 1,1,3-trichloropentafluoropropane was used. The results are shown in Table 6-7.

EXAMPLE 6-20

The fluorination reaction and the analysis of the reaction products were conducted in the same manner as in Example 6-1 except that 1,1,1-trichloropentafluoropropane was used. The results are shown in Table 6-7.

Table 6-7

| Example No. | 6-18 | 6-19 | 6-20 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 84 | 84 | 89 |
| Selectivity (%)<br>$CCl_3CF_2CF_3$<br>$CCl_2FCF_2CClF_2$<br>$CCl_2FCF_2CF_3$<br>$CClF_2CF_2CClF_2$<br>$CClF_2CF_2CF_3$<br>Others | <br>11<br>19<br>13<br>42<br>6<br>9 | <br><br><br>17<br>62<br>14<br>7 | <br><br><br>24<br><br>67<br>9 |

EXAMPLE 6-21

The fluorination reaction and the analysis of the reaction products were conducted in the same manner as in Example 6-1 except that 1,3-dichloro-hexafluoropropane was used. The results are shown in Table 6-8.

EXAMPLE 6-22

The fluorination reaction and the analysis of the reaction products were conducted in the same manner as in Example 6-1 except that 1,1-dichloro-hexafluoropropane was used. The results are shown in Table 6-8.

EXAMPLE 6-23

The fluorination reaction and the analysis of the reaction products were conducted in the same manner as in Example 6-1 except that l-chloro-heptafluoropropane was used. The results are shown in Table 6-8.

Table 6-8

| Example No. | 6-21 | 6-22 | 6-23 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 71 | 75 | 70 |
| Selectivity (%)<br>$CCIF_2CF_2CF_3$<br>$CF_3CF_2CF_3$<br>Others | 81<br>8<br>11 | 85<br>12<br>3 | <br>93<br>7 |

EXAMPLE 7-1

An Inconnel 600 U-shaped reaction tube having an inner diameter of 2.54 cm and a length of 100 cm packed with 200 ml of fluorination catalyst prepared in the same manner as in Preparation Example 1, was used as a fluorination reactor. To the reactor kept at a temperature of 300°C, gasified 1,1,3-trichloro-2,2,3,3-tetrafluoropropane (R-224ca), oxygen and hydrogen fluoride were fed in at a rate of 50 ml/minutes, 2 ml/minutes and 100 ml/minuts, respectively and reacted. The reaction products were collected in a trap at -78°C. The gas composition after removing acidic components from the collected product, was analyzed by gas chromatography and by $^{19}$F-NMR. The results are shown in Table 7-1.

EXAMPLE 7-2

The fluorination reaction and the analysis of the reaction products were conducted in the same manner as in Example 7-1 except that the catalyst prepared in Preparation Example'2 was used. The results are shown in Table 7-1.

EXAMPLE 7-3

The fluorination reaction and the analysis of the reaction products were conducted in the same manner as in Example 7-1 except that the catalyst prepared in Preparation Example 3 by using $Ba(NO_3)_2$ was used. The results are shown in Table 7-1.

EXAMPLE 7-4

The fluorination reaction and the analysis of the reaction products were conducted in the same manner as in Example 7-1 except that the catalyst prepared in Preparation Example 4 by using $Sr(NO_3)_2$ was used. The results are shown in Table 7-1.

Table 7-1

| Example No. | 7-1 | 7-2 | 7-3 | 7-4 |
|---|---|---|---|---|
| Reaction temp. (°C) | 300 | 300 | 300 | 300 |
| Ratio of HF/starting material (molar ratio) | 3 | 3 | 3 | 3 |
| Contact time (sec) | 20 | 20 | 20 | 20 |

**EP 0 407 622 B1**

Table 7-1   (continued)

| Example No. | 7-1 | 7-2 | 7-3 | 7-4 |
|---|---|---|---|---|
| Conversion (%) | 80 | 70 | 62 | 58 |
| Selectivity (%) $CCIF_2CF_2CHCIF$ $CF_3CF_2CHCl_2$ $CF_3C_2CHCIF$ $CCIF_2CF_2CHF_2$ Others | 85 5 3 2 5 | 70 24 1 3 2 | 57 35 1 2 5 | 65 29 0 1 5 |

EXAMPLE 7-5

The fluorination reaction and the analysis of the reaction products were conducted in the same manner as in Example 7-1 except that the catalyst prepared in Preparation Example 5 by using $Ca(NO_3)_2 \cdot 4H_2O$ was used. The results are shown in Table 7-2.

EXAMPLE 7-6

The fluorination reaction and the analysis of the reaction products were conducted in the same manner as in Example 7-1 except that the catalyst prepared in Preparation Example 6 by using $Mn(NO_3)_2 \cdot 4H_2O$ was used. The results are shown in Table 7-2.

EXAMPLE 7-7

The fluorination reaction and the analysis of the reaction products were conducted in the same manner as in Example 7-1 except that the catalyst prepared in Preparation Example 7 was used. The results are shown in Table 7-2.

EXAMPLE 7-8

The fluorination reaction and the analysis of the reaction products were conducted in the same manner as in Example 7-1 except that the catalyst prepared in Preparation Example 8 was used. The results are shown in Table 7-2.

Table 7-2

| Example No. | 7-5 | 7-6 | 7-7 | 7-8 |
|---|---|---|---|---|
| Reaction temp. (°C) | 300 | 300 | 300 | 300 |
| Ratio of HF/starting material (molar ratio) | 3 | 3 | 3 | 3 |
| Contact time (sec) | 20 | 20 | 20 | 20 |
| Conversion (%) | 57 | 72 | 51 | 54 |
| Selectivity (%) $CCIF_2CF_2CHCIF$ $CF_3CF_2CHCl_2$ $CF_3CF_2CHCIF$ $CCIF_2CF_2CHF_2$ Others | 61 34 0 1 4 | 71 21 2 3 3 | 68 27 1 2 2 | 70 23 1 2 4 |

EXAMPLE 7-9

The fluorination reaction and the analysis of the reaction products were conducted in the same manner as in Example 7-1 except that the catalyst prepared in Preparation Example 9 was used. The results are shown in Table 7-3.

11

EXAMPLE 7-10

The fluorination reaction and the analysis of the reaction products were conducted in the same manner as in Example 7-1 except that the catalyst prepared in Preparation Example 10 was used. The results are shown in Table 7-3.

EXAMPLE 7-11

The fluorination reaction and the analysis of the reaction products were conducted in the same manner as in Example 7-1 except that the catalyst prepared in Preparation Example 11 was used. The results are shown in Table 7-3.

EXAMPLE 7-12

The fluorination reaction and the analysis of the reaction products were conducted in the same manner as in Example 7-1 except that the catalyst prepared in Preparation Example 12 was used. The results are shown in Table 7-3.

Table 7-3

| Example No. | 7-9* | 7-10* | 7-11 | 7-12 |
|---|---|---|---|---|
| Reaction temp. (°C) | 300 | 300 | 300 | 300 |
| Ratio of HF/starting material (molar ratio) | 3 | 3 | 3 | 3 |
| Contact time (sec) | 20 | 20 | 20 | 20 |
| Conversion (%) | 50 | 47 | 50 | 53 |
| Selectivity (%)<br>$CCIF_2CF_2CHCIF$<br>$CF_3CF_2CHCl_2$<br>$CF_3CF_2CHCIF$<br>$CCIF_2CF_2CHF_2$<br>Others | <br>67<br>21<br>2<br>3<br>7 | <br>63<br>29<br>1<br>1<br>6 | <br>63<br>26<br>2<br>3<br>6 | <br>60<br>34<br>1<br>3<br>2 |

* Reference Example

EXAMPLE 7-13

The fluorination reaction and the analysis of the reaction products were conducted in the same manner as in Example 7-1 except that the catalyst prepared in Preparation Example 13 was used. The results are shown in Table 7-4.

EXAMPLE 7-14

The fluorination reaction and the analysis of the reaction products were conducted in the same manner as in Example 7-1 except that the catalyst prepared in Preparation Example 14 was used. The results are shown in Table 7-4.

Table 7-4

| Example No. | 7-13 | 7-14* |
|---|---|---|
| Reaction temp. (°C) | 300 | 300 |
| Ratio of HF/starting material (molar ratio) | 3 | 3 |
| Contact time (sec) | 20 | 20 |
| Conversion (%) | 41 | 46 |
| Selectivity (%)<br>$CCIF_2CF_2CHCIF$<br>$CF_3CF_2CHCl_2$<br>$CF_3CF_2CHCIF$<br>$CCIF_2CF_2CHF_2$ | <br>66<br>30<br>0<br>1 | <br>62<br>28<br>1<br>1 |

* Reference Example

Table 7-4   (continued)

| Example No. | 7-13 | 7-14* |
|---|---|---|
| Others | 3 | 8 |

* Reference Example

EXAMPLE 7-15

The fluorination reaction and the analysis of the reaction products were conducted in the same manner as in Example 7-1 except that 1,1,1,3,3-pentachloro-2,2-difluoropropane was used. The results are shown in Table 7-5.

EXAMPLE 7-16

The fluorination reaction and the analysis of the reaction products were conducted in the same manner as in Example 7-1 except that 1,1,3,3-tetrachloro-1,2,2-trifluoropropane was used. The results are shown in Table 7-5.

EXAMPLE 7-17

The fluorination reaction and the analysis of the reaction products were conducted in the same manner as in Example 7-1 except that 1,1,1,3-tetrachloro-2,2,3-trifluoropropane was used. The results are shown in Table 7-5.

EXAMPLE 7-18

The fluorination reaction and the analysis of the reaction products were conducted in the same manner as in Example 7-1 except that 1,1,3-trichloro-2,2,3,3-tetrafluoropropane was used. The results are shown in Table 7-5.

EXAMPLE 7-19

The fluorination reaction and the analysis of the reaction products were conducted in the same manner as in Example 7-1 except that 1,1,3-trichloro-1,2,2,3-tetrafluoropropane was used. The results are shown in Table 7-5.

Table 7-5

| Example No. | 7-15 | 7-16 | 7-17 | 7-18 | 7-19 |
|---|---|---|---|---|---|
| Reaction temp. (°C) | 300 | 300 | 300 | 300 | 300 |
| Ratio of HF/starting material (molar ratio) | 3 | 3 | 3 | 3 | 3 |
| Contact time (sec) | 20 | 20 | 20 | 20 | 20 |
| Conversion (%) | 74 | 73 | 79 | 75 | 80 |
| Selectivity (%) $CCl_2FCF_2CHClF$ | 6 | 9 | 10 | - | - |
| $CClF_2CF_2CHCl_2$ | 24 | 24 | - | - | - |
| $CClF_2CF_2CHClF$ | 53 | 50 | 54 | 68 | 85 |
| $CF_3CF_2CHCl_2$ | 5 | 7 | - | - | 5 |
| $CCl_2FCF_2CHF_2$ | - | - | 7 | - | - |
| $CClF_2CF_2CHF_2$ | 8 | 6 | 20 | 12 | 2 |
| $CF_3CF_2CHClF$ | 2 | 1 | 4 | 10 | 3 |
| Others | 2 | 3 | 5 | 5 | 5 |

EXAMPLE 7-20

The fluorination reaction and the analysis of the reaction products were conducted in the same manner as in Example 7-1 except that 1,1,1-trichloro-2,2,3,3-tetrafluoropropane was used. The results are shown in Table 7-6.

EXAMPLE 7-21

The fluorination reaction and the analysis of the reaction products were conducted in the same manner as in Example 7-1 except that 1,1-dichloro-2,2,3,3,3-pentafluoropropane was used. The results are shown in Table 7-6.

EXAMPLE 7-22

The fluorination reaction and the analysis of the reaction products were conducted in the same manner as in Example 7-1 except that 1,3-dichloro-1,1,2,2,3--pentafluoropropane was used. The results are shown in Table 7-6.

EXAMPLE 7-23

The fluorination reaction and the analysis of the reaction products were conducted in the same manner as in Example 7-1 except that 1,1-dichloro-1,2,2,3,3-pentafluoropropane was used. The results are shown in Table 7-6.

Table 7-6

| Example No. | 7-20 | 7-21 | 7-22 | 7-23 |
|---|---|---|---|---|
| Reaction temp. (°C) | 300 | 300 | 300 | 300 |
| Ratio of HF/starting material (molar ratio) | 3 | 3 | 3 | 3 |
| Contact time (sec) | 20 | 20 | 20 | 20 |
| Conversion (%) | 72 | 83 | 80 | 81 |
| Selectivity (%) $CCl_2FCF_2CHF_2$ $CClF_2CF_2CHF_2$ $CF_3CF_2CHClF$ $CF_3CF_2CHF_2$ Others | 12 71 - 8 9 | - - 78 15 7 | - 57 32 7 4 | - 74 - 23 3 |

EXAMPLE 7-24

The fluorination reaction and the analysis of the reaction products were conducted in the same manner as in Example 7-1 except that 1-chloro-1,2,2,3,3,3-hexafluoropropane was used. The results are shown in Table 7-7.

EXAMPLE 7-25

The fluorination reaction and the analysis of the reaction products were conducted in the same manner as in Example 7-1 except that 1-chloro-1,1,2,2,3,3-hexafluoropropane was used. The results are shown in Table 7-7.

Table 7-7

| Example No. | 7-24 | 7-25 |
|---|---|---|
| Reaction temp. (°C) | 300 | 300 |
| Ratio of HF/starting material (molar ratio) | 3 | 3 |
| Contact time (sec) | 20 | 20 |
| Conversion (%) | 83 | 77 |
| Selectivity (%) $CF_3CF_2CHF_2$ Others | 88 22 | 86 14 |

EXAMPLE 8-1

An Inconnel 600 U-shaped reaction tube having an inner diameter of 2.54 cm and a length of 100 cm packed with 200 ml of fluorination catalyst prepared in the same manner as in Preparation Example 1, was used as a fluorination reactor. To the reactor kept at a temperature of 350°C, gasified 1,3-dichloro-1,1,2,2-tetrafluoropropane, oxygen and hydrogen fluoride were fed in at a rate of 50 ml/minutes, 2 ml/minutes and 100 ml/minutes, respectively and reacted. The reaction products were collected in a trap cooled at -78°C. The gas composition, after removing acidic components from the collected product, was analyzed by gas chromatography or and $^{19}$F-NMR. The results are shown in Table 8-1.

EXAMPLE 8-2

The fluorination reaction and the analysis of the reaction products were conducted in the same manner as in Example 8-1 except that the catalyst prepared in Preparation Example 2 was used. The results are shown in Table 8-1.

EXAMPLE 8-3

The fluorination reaction and the analysis of the reaction products were conducted in the same manner as in Example 8-1 except that the catalyst prepared in Preparation Example 3 by using $Ba(NO_3)_2$ was used. The results are shown in Table 8-1.

Table 8-1

| Example No. | 8-1 | 8-2 | 8-3 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 85 | 72 | 64 |
| Selectivity (%)<br>$CClF_2CF_2CH_2F$<br>$CF_3CF_2CH_2Cl$<br>Others | 70<br>23<br>7 | 55<br>47<br>8 | 60<br>33<br>7 |

EXAMPLE 8-4

The fluorination reaction and the analysis of the reaction products were conducted in the same manner as in Example 8-1 except that the catalyst prepared in Preparation Example 4 by using $Sr(NO_3)_2$ was used. The results are shown in Table 8-2.

EXAMPLE 8-5

The fluorination-reaction and the analysis of the reaction products were conducted in the same manner as in Example 8-1 except that the catalyst prepared in Preparation Example 5 by using $Ca(NO_3)_2 \cdot 4H_2O$ was used. The results are shown in Table 8-2.

EXAMPLE 8-6

The fluorination reaction and the analysis of the reaction products were conducted in the same manner as in Example 8-1 except that the catalyst prepared in Preparation Example 6 by using $Mn(NO_3)_2 \cdot 4H_2O$ was used. The results are shown in Table 8-2.

Table 8-2

| Example No. | 8-4 | 8-5 | 8-6 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 61 | 63 | 76 |
| Selectivity (%) | | | |

Table 8-2 (continued)

| Example No. | 8-4 | 8-5 | 8-6 |
|---|---|---|---|
| $CClF_2CF_2CH_2F$ | 57 | 53 | 58 |
| $CF_3CF_2CH_2Cl$ | 36 | 41 | 36 |
| Others | 7 | 6 | 6 |

EXAMPLE 8-7

The fluorination reaction and the analysis of the reaction products were conducted in the same manner as in Example 8-1 except that the catalyst prepared in Preparation Example 7 was used. The results are shown in Table 8-3.

EXAMPLE 8-8

The fluorination reaction and the analysis of the reaction products were conducted in the same manner as in Example 8-1 except that the catalyst prepared in Preparation Example 8 was used. The results are shown in Table 8-3.

EXAMPLE 8-9

The fluorination reaction and the analysis of the reaction products were conducted in the same manner as in Example 8-1 except that the catalyst prepared in Preparation Example 9 was used. The results are shown in Table 8-3.

Table 8-3

| Example No. | 8-7 | 8-8 | 8-9* |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 38 | 42 | 40 |
| Selectivity (%)<br>$CClF_2CF_2CH_2F$<br>$CF_3CF_2CH_2Cl$<br>Others | 45<br>49<br>6 | 52<br>40<br>8 | 44<br>48<br>8 |

* Reference Example

EXAMPLE 8-10

The fluorination reaction and the analysis of the reaction products were conducted in the same manner as in Example 8-1 except that the catalyst prepared in Preparation Example 10 was used. The results are shown in Table 8-4.

EXAMPLE 8-11

The fluorination reaction and the analysis of the reaction products were conducted in the same manner as in Example 8-1 except that the catalyst prepared in Preparation Example 11 was used. The results are shown in Table 8-4.

EXAMPLE 8-12

The fluorination reaction and the analysis of the reaction products were conducted in the same manner as in Example 8-1 except that the catalyst prepared in Preparation Example 12 was used. The results are shown in Table 8-4.

Table 8-4

| Example No. | 8-10* | 8-11 | 8-12 |
|---|---|---|---|
| Reaction temp. (°C) | 300 | 300 | 300 |

* Reference Example

Table 8-4   (continued)

| Example No. | 8-10* | 8-11 | 8-12 |
|---|---|---|---|
| Ratio of HF/starting material (molar ratio) | 3 | 3 | 3 |
| Contact time (sec) | 20 | 20 | 20 |
| Conversion (%) | 67 | 41 | 42 |
| Selectivity (%)<br>$CClF_2CF_2CH_2F$<br>$CF_3CF_2CH_2Cl$<br>Others | 50<br>43<br>7 | 46<br>46<br>8 | 54<br>41<br>5 |

* Reference Example

EXAMPLE 8-13

The fluorination reaction and the analysis of the reaction products were conducted in the same manner as in Example 8-1 except that the catalyst prepared in Preparation Example 13 was used. The results are shown in Table 8-5.

EXAMPLE 8-14

The fluorination reaction and the analysis of the reaction products were conducted in the same manner as in Example 8-1 except that the catalyst prepared in Preparation Example 14 was used. The results are shown in Table 8-5.

Table 8-5

| Example No. | 8-13 | 8-14* |
|---|---|---|
| Reaction temp. (°C) | 300 | 300 |
| Ratio of HF/starting material (molar ratio) | 3 | 3 |
| Contact time (sec) | 20 | 20 |
| Conversion (%) | 62 | 60 |
| Selectivity (%)<br>$CClF_2CF_2CH_2F$<br>$CF_3CF_2CH_2Cl$<br>Others | 48<br>44<br>8 | 47<br>45<br>8 |

* Reference Example

EXAMPLE 8-15

The fluorination reaction and the analysis of the reaction products were conducted in the same manner as in Example 8-1 except that 1,1,3,3-tetrachloro-2,2-difluoropropane was used. The results are shown in Table 8-6.

EXAMPLE 8-16

The fluorination reaction and the analysis of the reaction products were conducted in the same manner as in Example 8-1 except that 1,1,1,3-tetrachloro-2,2-difluoropropane was used. The results are shown in Table 8-6.

EXAMPLE 8-17

The fluorination reaction and the analysis of the reaction products were conducted in the same manner as in Example 8-1 except that 1,1,3-trichloro-2,2,3-trifluoropropane was used. The results are shown in Table 8-6.

Table 8-6

| Example No. | 8-15 | 8-16 | 8-17 |
|---|---|---|---|
| Reaction temp. (°C) | 300 | 300 | 300 |

Table 8-6 (continued)

| Example No. | 8-15 | 8-16 | 8-17 |
|---|---|---|---|
| Ratio of HF/starting material (molar ratio) | 3 | 3 | 3 |
| Contact time (sec) | 20 | 20 | 20 |
| Conversion (%) | 74 | 70 | 81 |
| Selectivity (%) $CClF_2CF_2CH_2F$ $CF_3CF_2CH_2Cl$ Others | 50 43 7 | 46 46 8 | 54 41 5 |

EXAMPLE 8-18

The fluorination reaction and the analysis of the reaction products were conducted in the same manner as in Example 8-1 except that 1,1,3-trichloro-1,2,2-trifluoropropane was used. The results are shown in Table 8-7.

EXAMPLE 8-19

The fluorination reaction and the analysis of the reaction products were conducted in the same manner as in Example 8-1 except that 1,1,1-trichloro-2,2,3-trifluoropropane was used. The results are shown in Table 8-7.

EXAMPLE 8-20

The fluorination reaction and the analysis of the reaction products were conducted in the same manner as in Example 8-1 except that 1,1-dichloro-1,2,2,3-tetrafluoropropane was used. The results are shown in Table 8-7.

Table 8-7

| Example No. | 8-18 | 8-19 | 8-20 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 77 | 83 | 76 |
| Selectivity (%) $CCl_2FCF_2CH_2F$ $CClF_2CF_2CH_2Cl$ $CHClFCF_2CHF_2$ $CClF_2CF_2CH_2F$ $CF_3CF_2CH_2Cl$ $CHF_2CF_2CHF_2$ Others | 10 58 20 4 8 | 31 64 1 4 | 72 20 8 |

EXAMPLE 8-21

The fluorination reaction and the analysis of the reaction products were conducted in the same manner as in Example 8-1 except that 1,1-dichloro-2,2,3,3-tetrafluoropropane was used. The results are shown in Table 8-8.

EXAMPLE 8-22

The fluorination reaction and the analysis of the reaction products were conducted in the same manner as in Example 8-1 except that 1,3-dichloro-1,1,2,2-tetrafluoropropane was used. The results are shown in Table 8-8.

EXAMPLE 8-23

The fluorination reaction and the analysis of the reaction products were conducted in the same manner as in Example 8-1 except that 1,1-dichloro-1,2,2,3-tetrafluoropropane was used. The results are shown in Table 8-8.

Table 8-8

| Example No. | 8-21 | 8-22 | 8-23 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 64 | 69 | 72 |
| Selectivity (%) $CHClFCF_2CHF_2$ $CClF_2CF_3CH_2F$ $CF_3CF_2CH_2Cl$ $CHF_2CF_2CHF_2$ $CF_3CF_2CH_2F$ Others | 68 28 4 | 70 18 5 7 | 81 12 7 |

EXAMPLE 8-24

The fluorination reaction and the analysis of the reaction products were conducted in the same manner as in Example 8-1 except that 3-chloro-1,1,2,2,3-pentafluoropropane was used. The results are shown in Table 8-9.

EXAMPLE 8-25

The fluorination reaction and the analysis of the reaction products were conducted in the same manner as in Example 8-1 except that 3-chloro-1,1,1,1,2,2-pentafluoropropane was used. The results are shown in Table 8-9.

EXAMPLE 8-26

The fluorination reaction and the analysis of the reaction products were conducted in the same manner as in Example 8-1 except that 1-chloro-1,1,2,2,3-pentafluoropropane was used. The results are shown in Table 8-9.

Table 8-9

| Example No. | 8-24 | 8-25 | 8-26 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 63 | 60 | 64 |
| Selectivity (%) $CHF_2CF_2CHF_2$ $CF_3CF_2CH_2F$ Others | 90 10 | 94 6 | 92 8 |

EXAMPLE 9-1

An Inconnel 600 U-shaped reaction tube having an inner diameter of 2.54 cm and a length of 100 cm packed with 200 ml of fluorination catalyst prepared in the same manner as in Preparation Example 1, was used as a fluorination reactor. To the reactor kept at a temperature of 350°C, gasified 1,3-dichloro-1,1,2,2-tetrafluoropropane, oxygen and hydrogen fluoride were fed in at a rate of 50 ml/minutes, 2 ml/minutes and 100 ml/minutes, respectively and reacted.

The reaction products were collected in a trap cooled at -78°C. The gas composition, after removing acidic components from the collected product, was analyzed by gas chromatography and by 19F-NMR. The results are shown in Table 9-1.

EXAMPLE 9-2

The fluorination reaction and the analysis of the reaction products were conducted in the same manner as in Example 9-1 except that the catalyst prepared in Preparation Example 2 was used. The results are shown in Table 9-1.

EXAMPLE 9-3

The fluorination reaction and the analysis of the reaction products were conducted in the same manner as in Example 9-1 except that the catalyst prepared in Preparation Example 3 by using $Ba(NO_3)_2$ was used. The results are shown in Table 9-1.

Table 9-1

| Example No. | 9-1 | 9-2 | 9-3 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 83 | 70 | 61 |
| Selectivity (%)<br>$CHClFCF_2CH_2F$<br>$CHF_2CF_2CH_2F$<br>Others | 55<br>41<br>4 | 67<br>27<br>6 | 70<br>23<br>7 |

EXAMPLE 9-4

The fluorination reaction and the analysis of the reaction products were conducted in the same manner as in Example 9-1 except that the catalyst prepared in Preparation Example 4 by using $Sr(NO_3)_2$ was used. The results are shown in Table 9-2.

EXAMPLE 9-5

The fluorination reaction and the analysis of the reaction products were conducted in the same manner as in Example 9-1 except that the catalyst prepared in Preparation Example 5 by using $Ca(NO_3)_2 \cdot 4H_2O$ was used. The results are shown in Table 9-2.

EXAMPLE 9-6

The fluorination reaction and the analysis of the reaction products were conducted in the same manner as in Example 9-1 except that the catalyst prepared in Preparation Example 6 by using $Mn(NO_3)_2 \cdot 4H_2O$ was used. The results are shown in Table 9-2.

Table 9-2

| Example No. | 9-4 | 9-5 | 9-6 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 63 | 55 | 72 |
| Selectivity (%)<br>$CHClFCF_2CH_2F$<br>$CHF_2CF_2CH_2F$ | 66<br>30 | 71<br>22 | 59<br>35 |

Table 9-2   (continued)

| Example No. | 9-4 | 9-5 | 9-6 |
|---|---|---|---|
| Others | 4 | 7 | 6 |

EXAMPLE 9-7

The fluorination reaction and the analysis of the reaction products were conducted in the same manner as in Example 9-1 except that the catalyst prepared in Preparation Example 7 was used. The results are shown in Table 9-3.

EXAMPLE 9-8

The fluorination reaction and the analysis of the reaction products were conducted in the same manner as in Example 9-1 except that the catalyst prepared in Preparation Example 8 was used. The results are shown in Table 9-3.

EXAMPLE 9-9

The fluorination reaction and the analysis of the reaction products were conducted in the same manner as in Example 9-1 except that the catalyst prepared in Preparation Example 9 was used. The results are shown in Table 9-3.

Table 9-3

| Example No. | 9-7 | 9-8 | 9-9* |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 42 | 44 | 40 |
| Selectivity (%)<br>$CHClFCF_2CH_2F$<br>$CHF_2CF_2CH_2F$<br>Others | 73<br>20<br>7 | 68<br>25<br>7 | 76<br>18<br>6 |

* Reference Ex.

EXAMPLE 9-10

The fluorination reaction and the analysis of the reaction products were conducted in the same manner as in Example 9-1 except that the catalyst prepared in Preparation Example 10 was used. The results are shown in Table 9-4.

EXAMPLE 9-11

The fluorination reaction and the analysis of the reaction products were conducted in the same manner as in Example 9-1 except that the catalyst prepared in Preparation Example 11 was used. The results are shown in Table 9-4.

EXAMPLE 9-12

The fluorination reaction and the analysis of the reaction products were conducted in the same manner as in Example 9-1 except that the catalyst prepared in Preparation Example 12 was used. The results are shown in Table 9-4.

Table 9-4

| Example No. | 9-10* | 9-11 | 9-12 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |

* Reference Example

Table 9-4   (continued)

| Example No. | 9-10* | 9-11 | 9-12 |
|---|---|---|---|
| Conversion (%) | 63 | 42 | 48 |
| Selectivity (%) $CHClFCF_2CH_2F$ $CHF_2CF_2CH_2F$ Others | 65 30 5 | 72 21 7 | 74 21 5 |

* Reference Example

### EXAMPLE 9-13

The fluorination reaction and the analysis of the reaction products were conducted in the same manner as in Example 9-1 except that the catalyst prepared in Preparation Example 13 was used. The results are shown in Table 9-5.

### EXAMPLE 9-14

The fluorination reaction and the analysis of the reaction products were conducted in the same manner as in Example 9-1 except that the catalyst prepared in Preparation Example 14 was used. The results are shown in Table 9-5.

Table 9-5

| Example No. | 9-13 | 9-14* |
|---|---|---|
| Reaction temp. (°C) | 300 | 300 |
| Ratio of HF/starting material (molar ratio) | 3 | 3 |
| Contact time (sec) | 20 | 20 |
| Conversion (%) | 63 | 42 |
| Selectivity (%) $CHClFCF_2CH_2F$ $CHF_2CF_2CH_2F$ Others | 65 30 5 | 72 21 7 |

* Reference Ex.

### EXAMPLE 9-15

The fluorination reaction and the analysis of the reaction products were conducted in the same manner as in Example 9-1 except that 1,1,3-trichloro-2,2-difluoropropane was used. The results are shown in Table 9-6.

### EXAMPLE 9-16

The fluorination reaction and the analysis of the reaction products were conducted in the same manner as in Example 9-1 except that 1,1,1-trichloro-2,2-difluoropropane was used. The results are shown in Table 9-6.

### EXAMPLE 9-17

The fluorination reaction and the analysis of the reaction products were conducted in the same manner as in Example 9-1 except that 1,3-dichloro-1,2,2-trifluoropropane was used. The results are shown in Table 9-6.

### EXAMPLE 9-18

The fluorination reaction and the analysis of the reaction products were conducted in the same manner as in Example 9-1 except that 1,1-dichloro-2,2,3-trifluoropropane was used. The results are shown in Table 9-6.

Table 9-6

| Example No. | 9-15 | 9-16 | 9-17 | 9-18 |
|---|---|---|---|---|
| Reaction temp. (°C) | 300 | 300 | 300 | 300 |
| Ratio of HF/starting material (molar ratio) | 3 | 3 | 3 | 3 |
| Contact time (sec) | 20 | 20 | 20 | 20 |
| Conversion (%) | 84 | 77 | 80 | 68 |
| Selectivity (%) | | | | |
| $CHClFCF_2CH_2Cl$ | 19 | | | |
| $CHCl_2CF_2CH_2F$ | 22 | | | |
| $CH_3CF_2CCl_2F$ | | 21 | | |
| $CHClFCF_2CH_2F$ | 43 | | 53 | 55 |
| $CHF_2CF_2CH_2Cl$ | 9 | | 29 | |
| $CH_3CF_2CClF_2$ | | 60 | | |
| $CF_3CF_2CH_3$ | | 12 | 10 | 41 |
| Others | 7 | 7 | 8 | 9 |

EXAMPLE 9-19

The fluorination reaction and the analysis of the reaction products were conducted in the same manner as in Example 9-1 except that 1,1-dichloro-1,2,2-trifluoropropane was used. The results are shown in Table 9-7.

EXAMPLE 9-20

The fluorination reaction and the analysis of the reaction products were conducted in the same manner as in Example 9-1 except that 3-chloro-1,1,2,2-tetrafluoropropane was used. The results are shown in Table 9-7.

EXAMPLE 9-21

The fluorination reaction and the analysis of the reaction products were conducted in the same manner as in Example 9-1 except that 1-chloro-1,2,2,3-tetrafluoropropane was used. The results are shown in Table 9-7.

EXAMPLE 9-22

The fluorination reaction and the analysis of the reaction products were conducted in the same manner as in Example 9-1 except that 1-chloro-1,1,2,2-tetrafluoropropane was used. The results are shown in Table 9-7.

Table 9-7

| Example No. | 9-19 | 9-20 | 9-21 | 9-22 |
|---|---|---|---|---|
| Reaction temp. (°C) | 300 | 300 | 300 | 300 |
| Ratio of HF/starting material (molar ratio) | 3 | 3 | 3 | 3 |
| Contact time (sec) | 20 | 20 | 20 | 20 |
| Conversion (%) | 62 | 53 | 61 | 68 |
| Selectivity (%) | | | | |
| $CHClFCF_2CH_2F$ | 87 | | | |
| $CF_3CF_2CH_3$ | | | | 92 |
| $CHF_2CF_2CH_2F$ | 7 | 93 | 93 | |
| Others | 6 | 7 | 7 | 8 |

EXAMPLE 10-1

An Inconnel 600 U-shaped reaction tube having an inner diameter of 2.54 cm and a length of 100 cm packed with 200 ml of fluorination catalyst prepared in the same manner as in Preparation Example 1, was used as a fluorination reactor. To the reactor kept at a temperature of 350°C, gasified 1,3-dichloro-1,1,2,2-tetrafluoropropane, oxygen and hydrogen fluoride were fed in at a rate of 50 ml/minutes, 2 ml/minutes and 100 ml/minutes, respectively and reacted. The reaction products were collected in a trap cooled at -78°C. The gas composition, after removing acidic components from the collected product, was analyzed by gas chromatography or by $^{19}$F-NMR. The results are shown in Table 10-1.

EXAMPLE 10-2

The fluorination reaction and the analysis of the reaction products were conducted in the same manner as in Example 10-1 except that the catalyst prepared in Preparation Example 2 was used. The results are shown in Table 10-1.

EXAMPLE 10-3

The fluorination reaction and the analysis of the reaction products were conducted in the same manner as in Example 10-1 except that the catalyst prepared in Preparation Example 3 by using $Ba(NO_3)_2$ was used. The results are shown in Table 10-1.

Table 10-1

| Example No. | 10-1 | 10-2 | 10-3 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 80 | 74 | 68 |
| Selectivity (%)<br>$CHClFCF_2CH_3$<br>$CHF_2CF_2CH_3$<br>Others | 82<br>14<br>6 | 80<br>13<br>7 | 85<br>11<br>4 |

EXAMPLE 10-4

The fluorination reaction and the analysis of the reaction products were conducted in the same manner as in Example 10-1 except that the catalyst-prepared in Preparation Example 4 by using $Sr(NO_3)_2$ was used. The results are shown in Table 10-2.

EXAMPLE 10-5

The fluorination reaction and the analysis of the reaction products were conducted in the same manner as in Example 10-1 except that the catalyst prepared in Preparation Example 5 by using $Ca(NO_3)_2 \cdot 4H_2O$ was used. The results are shown in Table 10-2.

EXAMPLE 10-6

The fluorination-reaction and the analysis of the reaction products were conducted in the same manner as in Example 10-1 except that the catalyst prepared in Preparation Example 6 by using $Mn(NO_3)_2 \cdot 4H_2O$ was used. The results are shown in Table 10-2.

Table 10-2

| Example No. | 10-4 | 10-5 | 10-6 |
|---|---|---|---|
| Reaction temp. (°C) | 300 | 300 | 300 |

Table 10-2   (continued)

| Example No. | 10-4 | 10-5 | 10-6 |
|---|---|---|---|
| Ratio of HF/starting material (molar ratio) | 3 | 3 | 3 |
| Contact time (sec) | 20 | 20 | 20 |
| Conversion (%) | 60 | 63 | 75 |
| Selectivity (%)<br>$CHClFCF_2CH_3$<br>$CHF_2CF_2CH_3$<br>Others | 88<br>7<br>5 | 84<br>8<br>8 | 86<br>9<br>5 |

EXAMPLE 10-7

The fluorination reaction and the analysis of the reaction products were conducted in the same manner as in Example 10-1 except that the catalyst prepared in Preparation Example 7 was used. The results are shown in Table 10-3.

EXAMPLE 10-8

The fluorination reaction and the analysis of the reaction products were conducted in the same manner as in Example 10-1 except that the catalyst prepared in Preparation Example 8 was used. The results are shown in Table 10-3.

EXAMPLE 10-9

The fluorination reaction and the analysis of the reaction products were conducted in the same manner as in Example 10-1 except that the catalyst prepared in Preparation Example 9 was used. The results are shown in Table 10-3.

Table 10-3

| Example No. | 10-7 | 10-8 | 10-9* |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 35 | 40 | 36 |
| Selectivity (%)<br>$CHClFCF_2CH_3$<br>$CHF_2CF_2CH_3$<br>Others | 90<br>3<br>7 | 93<br>2<br>5 | 92<br><br>8 |

* Reference Ex.

EXAMPLE 10-10

The fluorination reaction and the analysis of the reaction products were conducted in the same manner as in Example 10-1 except that the catalyst prepared in Preparation Example 10 was used. The results are shown in Table 10-4.

EXAMPLE 10-11

The fluorination reaction and the analysis of the reaction products were conducted in the same manner as in Example 10-1 except that the catalyst prepared in Preparation Example 11 was used. The results are shown in Table 10-4.

EXAMPLE 10-12

The fluorination reaction and the analysis of the reaction products were conducted in the same manner as in Example 10-1 except that the catalyst prepared in Preparation Example 12 was used. The results are shown in Table 10-4.

Table 10-4

| Example No. | 10-10* | 10-11 | 10-12 |
|---|---|---|---|
| Reaction temp. (°C) | 300 | 300 | 300 |
| Ratio of HF/starting material (molar ratio) | 3 | 3 | 3 |
| Contact time (sec) | 20 | 20 | 20 |
| Conversion (%) | 71 | 38 | 38 |
| Selectivity (%)<br>$CHClFCF_2CH_3$<br>$CHF_2CF_2CH_3$<br>Others | 83<br>9<br>8 | 91<br><br>9 | 92<br><br>8 |

* Reference Ex.

EXAMPLE 10-13

The fluorination reaction and the analysis of the reaction products were conducted in the same manner as in Example 10-1 except that the catalyst prepared in Preparation Example 13 was used. The results are shown in Table 10-5.

EXAMPLE 10-14

The fluorination reaction and the analysis of the reaction products were conducted in the same manner as in Example 10-1 except that the catalyst prepared in Preparation Example 14 was used. The results are shown in Table 10-5.

Table 10-5

| Example No. | 10-13 | 10-14* |
|---|---|---|
| Reaction temp. (°C) | 300 | 300 |
| Ratio of HF/starting material (molar ratio) | 3 | 3 |
| Contact time (sec) | 20 | 20 |
| Conversion (%) | 65 | 61 |
| Selectivity (%)<br>$CHClFCF_2CH_3$<br>$CHF_2CF_2CH_3$<br>Others | 90<br>2<br>8 | 93<br><br>7 |

* Reference Ex.

EXAMPLE 10-15

The fluorination reaction and the analysis of the reaction products were conducted in the same manner as in Example 10-1 except that 1,3-dichloro-2,2-difluoropropane was used. The results are shown in Table 10-6.

EXAMPLE 10-16

The fluorination reaction and the analysis of the reaction products were conducted in the same manner as in Example 10-1 except that 1,1-dichloro-2,2-difluoropropane was used. The results are shown in Table 10-6.

EXAMPLE 10-17

The fluorination reaction and the analysis of the reaction products were conducted in the same manner as in Example 10-1 except that 1-chloro-2,2,3-trifluoropropane was used. The results are shown in Table 10-6.

EXAMPLE 10-18

The fluorination reaction and the analysis of the reaction products were conducted in the same manner as in Example 10-1 except that 1-chloro-1,2,2-trifluoropropane was used. The results are shown in Table 10-6.

Table 10-6

| Example No. | 10-15 | 10-16 | 10-17 | 10-18 |
|---|---|---|---|---|
| Reaction temp. (°C) | 300 | 300 | 300 | 300 |
| Ratio of HF/starting material (molar ratio) | 3 | 3 | 3 | 3 |
| Contact time (sec) | 20 | 20 | 20 | 20 |
| Conversion (%) | 77 | 53 | 64 | 60 |
| Selectivity (%) $CH_2FCF_2CH_2Cl$ $CH_3CF_2CHClF$ $CH_2FCF_2CH_2F$ $CH_3CF_2CHF_2$ Others | 58 34 8 | 80 14 6 | 97 3 | 91 9 |

**Claims**

1. A process for producing a 2,2-difluoropropane of the following formula (2), which comprises fluorinating a chlorine-containing 2,2-difluoropropane of the following formula (1) with hydrogen fluoride in a gas phase, in the presence of a fluorination catalyst composition comprising oxides of the following elements in combination:

   Cr/Mg or Al/Cr/Mg or Al/Cr/Ba or Al/Cr/Sr or Al/Cr/Ca or Al/Cr/Mn or Al/Fe or Cr/Fe or Al/Mn or Al/Ni orAl/Co

   said fluorination being conducted at a reaction temperature of from 150°C - 550°C,

   (1) $\quad C_3H_aCl_bF_c$

   (2) $\quad C_3H_aCl_{b-x}F_{c+x}$

   wherein a, b, c and x are integers satisfying the following conditions
   $a \geq 0, b \geq 1, c \geq 2, x \geq 1,$
   $a + b + c = 8.$

2. The process according to Claim 1, wherein the chlorine-containing 2,2-fluoropropane of the formula (1) is $C_3Cl_{6-m1}F_{2+m1}$ ($0 \leq m^1 \leq 5$), and the 2,2-difluoropropane of the formula (2) is $C_3Cl_{6-n1}F_{2+n1}$ ($1 \leq n^1 \leq b, m^1 < n^1$).

3. The process according to Claim 1, wherein the chlorine-containing 2,2-fluoropropane of the formula (1) is $C_3HCl_{5-m2}F_{2+m2}$ ($0 \leq m^2 \leq 4$), and the 2,2-difluoropropane of the formula (2) is $C_3HCl_{5-n2}F_{2+n2}$ ($1 \leq n^2 \leq 5, m^2 < n^2$).

4. The process according to Claim 1, wherein the chlorine-containing 2,2-fluoropropane of the formula (1) is $C_3H_2Cl_{4-m3}F_{2+m3}$ ($0 \leq m^3 \leq 3$), and the 2,2-diflucropropane of the formula (2) is $C_3H_2Cl_{4-n3}F_{2+n3}$ ($1 \leq n^3 \leq 4, m^3 < n^3$).

5. The process according to Claim 1, wherein the chlorine-containing 2,2-fluoropropane of the formula (1) is

$C_3H_3Cl_{3-m4}F_{2+m4}$ ($0 \leq m^4 \leq 2$), and the 2,2-difluoropropane of the formula (2) is $C_3H_3Cl_{3-n4}F_{2+n4}$ ($1 \leq n^4 \leq 3$, $m^4 < n^4$).

6. The process according to Claim 1, wherein the chlorine-containing 2,2-fluoropropane of the formula (1) is $C_3H_4Cl_{2-m5}F_{2+m5}$ ($0 \leq m^5 \leq 1$), and the 2,2-difluoropropane of the formula (2) is $C_3H_4Cl_{2-n5}F_{2+n5}$ ($1 \leq n^5 \leq 2$, $m^5 < n^5$).

**Patentansprüche**

1. Verfahren zur Herstellung eines 2,2-Difluorpropans der folgenden Formel (2), umfassend eine Fluorierung eines Chlor-enthaltenden 2,2-Difluorpropans der folgenden Formel (1) mit Fluorwasserstoff in der Gasphase in Gegenwart einer Fluorierungskatalysatorzusammensetzung, umfassend eine Kombination aus Oxiden der folgenden Elemente:

   Cr/Mg oder Al/Cr/Mg oder Al/Cr/Ba oder Al/Cr/Sr oder Al/Cr/Ca oder Al/Cr/Mn oder Al/Fe oder Cr/Fe oder Al/Mn oder Al/Ni oder Al/Co,

   wobei die Fluorierung durchgeführt wird bei einer Reaktionstemperatur von 150° bis 550°C,

$$(1) \qquad C_3H_aCl_bF_c$$

$$(2) \qquad C_3H_aCl_{b-x}F_{c+x},$$

   wobei a, b, c und x ganze Zahlen bedeuten, die folgende Bedingungen erfüllen
   $a \geq 0$, $b \geq 1$, $c \geq 2$, $x \geq 1$,
   a+b+c=8.

2. Verfahren gemäß Anspruch 1, wobei das Chlor-enthaltende 2,2-Fluorpropan der Formel (1) $C_3Cl_{6-m1}F_{2+m1}$ ($0 \leq m^1 \leq 5$) ist, und wobei das 2,2-Difluorpropan der Formel (2) $C_3Cl_{6-n1}F_{2+n1}$ ($1 \leq n^1 \leq b$, $m^1 < n^1$) ist,

3. Verfahren gemäß Anspruch 1, wobei das Chlor-enthaltende 2,2-Fluorpropan der Formel (1) $C_3HCl_{5-m2}F_{2+m2}$ ($0 \leq m^2 \leq 4$) ist, und wobei das 2,2-Difluorpropan der Formel (2) $C_3HCl_{5-n2}F_{2+n2}$ ($1 \leq n^2 \leq 5$, $m^2 < n^2$) ist.

4. Verfahren gemäß Anspruch 1, wobei das Chlor-enthaltende 2,2-Fluorpropan der Formel (1) $C_3H_2Cl_{4-m3}F_{2+m3}$ ($0 \leq m^3 \leq 3$) ist, und wobei das 2,2-Difluorpropan der Formel (2) $C_3H_2Cl_{4-n3}F_{2+n3}$ ($1 \leq n^3 < 4$, $m^3 < n^3$) ist.

5. Verfahren gemäß Anspruch 1, wobei das Chlor-enthaltende 2,2-Fluorpropan der Formel (1) $C_3H_3Cl_{3-m4}F_{2+m4}$ ($0 \leq m^4 \leq 2$) ist, und wobei das 2,2-Difluorpropan der Formel (2) $C_3H_3Cl_{3-n4}F_{2+n4}$ ($1 \leq n^4 \leq 3$, $m^4 < n^4$) ist.

6. Verfahren gemäß Anspruch 1, wobei das Chlor-enthaltende 2,2-Fluorpropan der Formel (1) $C_3H_4Cl_{2-m5}F_{2+m5}$ ($0 \leq m^5 \leq 1$) ist, und wobei das 2,2-Difluorpropan der Formel (2) $C_3H_4Cl_{2-n5}F_{2+n5}$ ($1 \leq n^5 \leq 2$, $m^5 < n^5$) ist.

**Revendications**

1. Procédé de préparation d'un 2,2-difluoropropane de la formule suivante (2), qui comprend la fluoration d'un 2,2-difluoropropane contenant du chlore de la formule suivante (1) par du fluorure d'hydrogène en phase gazeuse, en présence d'une composition catalytique de fluoration comprenant des oxydes des éléments suivants en combinaison :

   Cr/Mg ou Al/Cr/Mg ou Al/Cr/Ba ou Al/Cr/Sr ou Al/Cr/Ca ou Al/Cr/Mn ou Al/Fe ou Cr/Fe ou Al/Mn ou Al/Ni ou Al/Co

   ladite fluoration étant conduite à une température de réaction de 150°C - 550°C,

$$(1) \qquad C_3H_aCl_bF_c$$

$$(2) \qquad C_3H_aCl_{b-x}F_{c+x}$$

où a, b, c et x sont des entiers satisfaisant les conditions suivantes :

$a \geq 0$, $b \geq 1$, $c \geq 2$, $x \geq 1$,

$a + b + c = 8$.

2. Procédé selon la revendication 1, dans lequel le 2,2-fluoropropane contenant du chlore de la formule (1) est $C_3Cl_{6-m^1}F_{2+m^1}$ ($0 \leq m^1 \leq 5$), et le 2,2-difluoropropane de la formule (2) est $C_3Cl_{6-n^1}F_{2+n^1}$ ($1 \leq n^1 \leq b$, $m^1 < n^1$).

3. Procédé selon la revendication 1, dans lequel le 2,2-fluoropropane contenant du chlore de la formule (1) est $C_3HCl_{5-m^2}F_{2+m^2}$ ($0 \leq m^2 \leq 4$), et le 2,2-difluoropropane de la formule (2) est $C_3HCl_{5-n^2}F_{2+n^2}$ ($1 \leq n^2 \leq 5$, $m^2 < n^2$).

4. Procédé selon la revendication 1, dans lequel le 2,2-fluoropropane contenant du chlore de la formule (1) est $C_3H_2Cl_{4-3}F_{2+m^3}$ ($0 \leq m^3 \leq 3$), et le 2,2-difluoropropane de la formule (2) est $C_3H_2Cl_{4-n^3}F_{2+n^3}$ ($1 \leq n^3 \leq 4$, $m^3 < n^3$).

5. Procédé selon la revendication 1, dans lequel le 2,2-fluoropropane contenant du chlore de la formule (1) est $C_3H_3Cl_{3-m^4}F_{2+m^4}$ ($0 \leq m^4 \leq 2$), et le 2,2-difluoropropane de la formule (2) est $C_3H_3Cl_{3-n^4}F_{2+n^4}$ ($1 \leq n^4 \leq 3$, $m^4 < n^4$).

6. Procédé selon la revendication 1, dans lequel le 2,2-fluoropropane contenant du chlore de la formule (1) est $C_3H_4Cl_{2-5}F_{2+m^5}$ ($0 \leq m^5 \leq 1$), et le 2,2-difluoropropane de la formule (2) est $C_3H_4Cl_{2-n^5}F_{2+n^5}$ ($1 \leq n^5 \leq 2$, $m^5 < n^5$).